(19) Europäisches Patentamt
European Patent Office
Office européen des brevets

(11) Veröffentlichungsnummer: **0 208 962 B1**

# (12) EUROPÄISCHE PATENTSCHRIFT

(45) Veröffentlichungstag der Patentschrift: **02.03.94**

(51) Int. Cl.[5]: **A61K 31/62**, //(A61K31/62, 31:52,31:557)

(21) Anmeldenummer: **86108697.3**

(22) Anmeldetag: **26.06.86**

## (54) Kombinationspräparate aus Hemmstoffen der Thrombozytenaggregation und deren Verwendung.

(30) Priorität: **05.07.85 DE 3524051**

(43) Veröffentlichungstag der Anmeldung:
**21.01.87 Patentblatt 87/04**

(45) Bekanntmachung des Hinweises auf die Patenterteilung:
**02.03.94 Patentblatt 94/09**

(84) Benannte Vertragsstaaten:
**AT BE CH DE FR GB IT LI LU NL SE**

(56) Entgegenhaltungen:
**EP-A- 0 169 466**

**PROSTAGLANDINS, Band 28, Nr. 4, Okfober 1984, Seiten 443-453, Los Altos, California, US; S.J. KONTUREK et al.: "Comparison of gastric and antistinal intisecretory and protective effects of prostacyclin and its stable thia-imino-analogue (hoe 892) in conscious rats"**

(73) Patentinhaber: **HOECHST AKTIENGESELLSCHAFT**

**D-65926 Frankfurt(DE)**

(72) Erfinder: **Weithmann, Klaus Ulrich, Dr.**
**Am Domherrnwald 18**
**D-6238 Hofheim am Taunus(DE)**

**EP 0 208 962 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es sind bereits Arzneimittel mit antithrombotischen und antiaggregatorischen Wirkungen bekannt. So zeigt O-Acetylsalicylsäure in der vorbeugenden Behandlung gegen Thrombosen und Schlaganfälle klinisch nachweisbare Effekte.

Das 1-(5-Oxohexyl)-3,7-dimethylxanthin (Pentoxifyllin) wird bekanntlich als pharmazeutisches Mittel zur Verbesserung der Fließfähigkeit des Blutes eingesetzt.

Es ist bereits bekannt, daß der physiologische Arachidonsäure-Metabolit Prostacyclin der Formel IV

(IV)

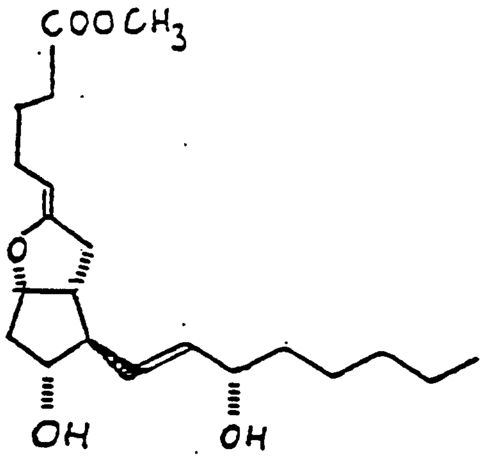

eine überaus wirksame antiaggregatorsiche Substanz ist. Als Wirkungsmechanismus ist beschrieben worden, daß Prostacyclin das zyklische 3',5'-Adenosinmonophosphat (cyclo AMP) in den Thrombozyten stimuliert (Nature (London) 292 (1981) 364).

Leider ist die medizinische Anwendung von Prostacylin wegen seiner kurzen Halbwerszeit bei physiologischem pH stark eingeschränkt. Noch instabiler ist das Molekül bei saurem pH, so daß auch eine therapeutisch erfolgreiche orale Anwendung nicht möglich ist (Prostaglandins, 15 (1978) 943). Es sind aber stabile Analoga des Prostacyclins, wie Carbecycline (J. Org. Chem. 46 (1981), 1954, und Heteroiminoprostacycline (s. EP 34778) beschrieben worden, bei denen die hydrolyseempfindliche Enoläther-Struktur im Prostacyclin-Molekül durch hydrolyseunempfindliche Strukturelemente ersetzt worden ist. Das Heteroiminoprostacyclin 2-Aza-3-(4-carboxymethyl-1-thiabutyl)-6-β-(3-α-hydroxy-1-octenyl)-7-α-hydroxy-bicyclo(3.3.0)-octen-2) der Formel II ist unter dem Namen Tilsuprost bekannt.

Es ist bekannt, daß die Heteroiminoprostacycline biologisch sehr aktiv sind. Sie wirken unter anderem blutgefäßerweiternd, antithrombotisch, inhibieren die Thrombozyetenaggregation und verhindern den Bronchospasmus. Wegen dieser wertvollen medizinischen Eigenschaften können Vertreter dieser Stoffklasse u.a. als Medikamente zur Blutdrucksenkung, als antiaggregatorisch und antithrombotisch wirksame Substanzen, zur Verbesserung der Fließeigenschaften des Blutes sowie als Bronchospasmolytika, aber auch als Cytostatika eingesetzt werden.

Durch eigene Versuche konnte bestätigt werden, daß die antiaggregatorischen Wirkstärken in vitro für Tilsuprost in der gleichen Größenordnung liegen wie für Prostacyclin. In einer Reihe von eigenen biologischen Untersuchungen wurde aber auch gefunden, daß sich Prostacyclin und Heteroiminoprostacycline vielfach höchst unterschiedlich verhalten. So hat sich z.B. Überraschenderweise gezeigt, daß Tilsuprost im Gegensatz zu Prostacyclin keine signifikante stimulierende Wirkung auf den cyclo-AMP-Spiegel in Humanthrombozyten besitzt. Mit anderen Worten üben Heteroiminoprostacycline, wie Tilsuprost, und das natürliche Prostacyclin voneinander völlig verschiedene biochemische und pharmakologische-Wirkungen aus (s. Tab. 2).

Kombinationspräparate aus Heteroiminoprostacyclinen und gewissen Xanthinen (siehe unten) Zeigen über-additive Effekte.

Die Erfindung bezieht sich nun auf Heteroiminoprostacycline der Formel I (siehe Formelblatt). Hierunter sind im folgenden solche zu verstehen, bei denen bedeutet:

X ein Sauerstoff- oder Schwefelatom oder eine NH-Gruppe,

Y einen geradkettigen aliphatischen Kohlenwasserstoffrest mit 2 - 6 Kohlenstoffatomen oder einen cycloaliphatischen Kohlenwasserstoffrest mit 3 - 6 Kohlenstoffatomen im Ring, die

höchstens eine Mehrfachbindung enthalten können,

Z einen Rest der Formel $-CO_2R^1$ oder $-CH_2OH$, wobei $R^1$ Wasserstoff, einen geradkettigen oder verzweigten Alkylrest mit bis zu 8 Kohlenstoffatomen, einen geradkettigen oder verzweigten einfach olefinisch-ungesättigten aliphatischen Kohlenwasserstoffrest mit 3 - 8 Kohlenstoffatomen, einen cycloaliphatischen Kohlenwasserstoffrest, der 3 - 7 Kohlenstoffatome im Ring und höchstens eine Mehrfachbindung enthält, oder einen araliphatischen Kohlenwasserstoffrest mit 6 - 9 Kohlenstoffatomen oder ein geeignetes pharmazeutisch verträgliches Metall- oder Ammoniumkation,

$R^2$ einen geradkettigen oder verzweigten aliphatischen Kohlenwasserstoffrest mit 4 bis 6 Kohlenstoffatomen, der höchstens eine Mehrfachbindung enthalten kann,

$R^3$ und $R^4$ entweder Wasserstoff oder gleiche oder verschiedene, unter neutralen oder basischen Bedingungen leicht abspaltbare Schutzgruppen.

Der Begriff "Mehrfachbindung" umfaßt sowohl acetylenische als auch olefinische Bindungen; letztere sind bevorzugt. Die Metallkationen $R^1$ können z.B. solche der Alkalimetalle, wie Lithium, Natrium und Kalium, und der Erdalkalimetalle, wie Magnesium und Calcium, aber auch kationische Formen anderer Metalle, wie Aluminium, Zink und Eisen sein. Pharmakologisch verträgliche Ammoniumkationen sind solche, die sich von Ammoniak oder primären, sekundären oder tertiären Aminen ableiten, wie der Alkylamine, z.B. Mono-, Di- und Trimethyl-, bzw. -äthyl-, -propyl-, -isopropyl-, -butyl-, -isobutyl-, -t-butyl-, sowie N-(Methylhexyl)-, N-Methyl-N-hexyl-, Benzyl-, β-Phenyläthylamin, Äthylendiamin, Diäthylentriamin, Pyrrolidin, Piperidin, Morpholin, Piperazin, Mono-, Di- und Triäthanolamin, Äthyldiäthanolamin, N-Butyläthanolamin, Tris-(hydroxymethyl)-aminomethan und dergleichen. Geeignete Ammoniumsalze sind z.B. die basischen Aminsalze des Lysins und des Arginins. Geeignete pharmakologisch verträgliche quaternäre Ammoniumkationen sind z.B. das Tetramethylammonium, Tetraäthylammonium und das Benzyltrimethylammonium.

Gegenstand der Erfindung ist ein Kombinationspräparat, enthaltend

A) ein Heteroiminoprostacyclin der Formel I

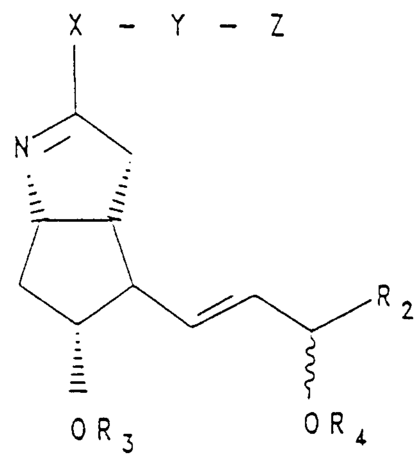

(I)

worin bedeutet

X ein Sauerstoff- oder Schwefelatom oder eine NH-Gruppe,

Y einen geradkettigen aliphatischen Kohlenwasserstoffrest mit 2 - 6 Kohlenstoffatomen oder einen cycloaliphatischen Konlenwasserstoffrest mit 3 - 6 Kohlenstoffatomen im Ring, die höchstens eine Mehrfachbindung enthalten können,

Z einen Rest der Formel $-CO_2R^1$ oder $-CH_2OH$, wobei $R^1$ Wasserstoff, einen Alkylrest mit bis zu 8 Kohlenstoffatomen, einen einfach olefinisch-ungesättigten aliphatischen Kohlenwasserstoffrest mit 3 - 8 Kohlenstoffatomen, einen cycloaliphatischen Kohlenwasserstoffrest, der 3 - 7 Kohlenstoffatome im Ring und höchstens eine Mehrfachbindung enthält, oder einen araliphatischen Kohlenwasserstoffrest mit 6 - 9 Kohlenstoffatomen oder ein pharmazeutisch verträgliches Metall- oder Ammoniumkation darstellt,

$R^2$ einen aliphatischen Kohlenwasserstoffrest mit 4 bis 6 Kohlenstoffatomen, der höchstens eine Mehrfachbindung enthalten kann,

$R^3$ und $R^4$ Wasserstoff oder gleiche oder verschiedene, unter neutralen oder basischen Bedingungen leicht abspaltbare Schutzgruppen,

und

B) wenigstens ein Xanthin der Formel (III) oder (IIIa),

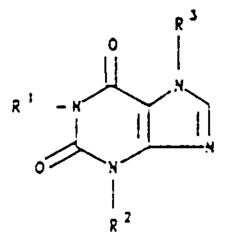
(III)

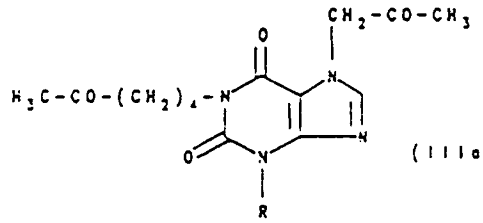
(IIIa)

worin einer der Reste $R^1$ und $R^3$ eine geradkettige Alkyl-, ($\omega$-1)-Oxoalkyl- oder ($\omega$-1)-Hydroxyalkylgruppe - die in $\omega$- oder ($\omega$-2)-Stellung eine weitere Hydroxylgruppe enthalten kann - mit 3 bis 8 C-Atomen und die beiden anderen Reste $R^2$ und $R^3$ oder $R^1$ und $R^2$ Alkylgruppen mit 1 bis 8 C-Atomen in der Position von $R^1$ bzw. $R^3$ und 1 bis 4 C-Atomen in der Position von $R^2$ darstellen und in Formel (IIIa) R für einen Alkylrest mit 1 bis 4 C-Atomen steht, oder eine Prodrug-Form der Oxoalkylxanthine der Formen (III) oder (IIIa) oder der Hydroxyalkylxanthine der Formel (III), oder einen Metabolit eines Xanthins der Formel (III) oder (IIIa),

wobei das Gewichtsverhältnis der Komponente A zur Komponente B zwischen 1: 8000 und 1: 10 liegt, mit oder ohne einen pharmazeutischen Träger, zur Behandlung von Krankheiten, die durch gestörte Blutfunktionen oder Blutbestandteile, insbesondere Thrombozyten oder Erythrozythen, oder durch gestörte physiologische Wechselwirkungen zwischen den Blutbestandteilen und der Blutgefäßwand verursacht oder gekennzeichnet sind.

Ein weiterer Gegenstand der Erfindung ist ein Kombinationspräparat, das dadurch gekennzeichnet, daß es als Komponente A das Heteroiminoprostacyclin die Formel V aufweist

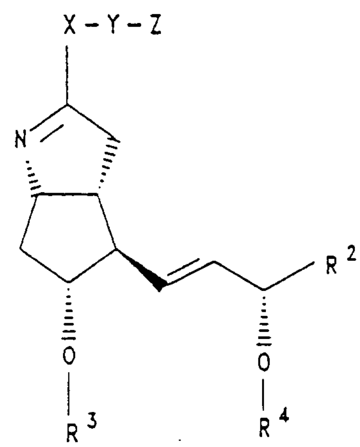
(V)

wobei X, Y, Z, $R^2$, $R^3$ oder $R^4$ die in Formel I genannte Definitionen haben, und als Komponente B das Xanthinderivat der Formel III

wobei

a) die Summe der C-Atome aller Substituenten 3 bis 10 beträgt,

b) $R^1$ oder $R^3$ ein Rest mit 6 C-Atomen ist oder

c) $R^1$ oder $R^3$ ein Rest mit 6 C-Atomen ist, der in ($\omega$-1)-Stellung mit einer Hydroxy- oder Oxogruppe substituiert ist,

enthält.

Ein weiterer Gegenstand der Erfindung ist ein Kombinationspräparat, das dadurch gekennzeichnet ist, daß es als Komponente A das Tilsuprost der Formel II

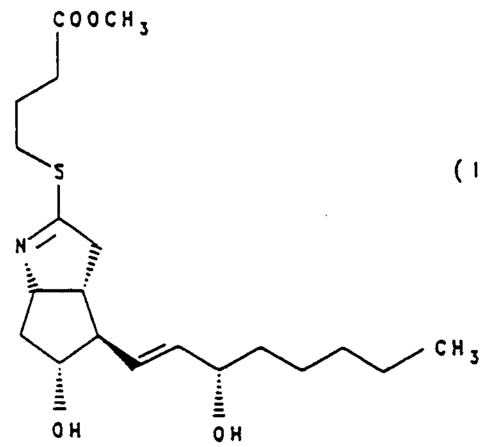

(II)

enthält.

Ein weiterer Gegenstand der Erfindung ist ein Kombinationspräparat, das dadurch gekennzeichnet ist, daß das Gewichtsverhältnis der Komponente A zu der Komponente B zwischen 1: 2000 und 1 : 20 liegt, insbesondere zwischen 1: 500 und 1: 50 liegt.

Gegenstand der Erfindung sind auch die Verwendung von Kombinationen der Komponenten A) und B) zur Behandlung der genannten Krankheiten und ein Verfahren zur Herstellung von pharmazeutischen Zubereitungen.

Erfindungsgemäß können also die pharmakologischen Wirkungen gerade der Heteroiminoprostacycline durch Zusatz der Xanthine überadditiv stimuliert werden (s. Tabelle 3), und zwar weit über die berechnete Summe der Wirkungen der Einzelsubstanzen hinaus.

Bevorzugt sind solche Xanthinderivate der Formel (III), bei denen die Summe der C-Atome aller Substituenten mindestens 3 und höchstens 10 beträgt. Außergewöhnlich potente Wirkungssteigerungen werden, wie unten näher ausgeführt wird, mit solchen Xanthinderivaten erhalten, bei denen sich in der Position von $R^1$ oder $R^3$ eine Gruppe mit 6 C-Atomen befindet, die bis zu einer Hydroxy- bzw. Oxogruppe in ($\omega$ -1)-Stellung enthalten kann, also eine Hexyl-, 5-Oxohexyl- oder 5-Hydroxyhexylgruppe, d.h. insbesondere 1-Hexyl-3,7-dimethylxanthin (Pentifyllin), 1-(5-Hydroxyhexyl)-3,7-dimethylxanthin, 1-(5-Oxohexyl)-3,7-dimethyl-xanthin, 1,3-Dimethyl-7-(5-hydroxyhexyl)-xanthin, 1,3-Dimethyl-7-(5-oxohexyl)-xanthin, 1-(5-Hydroxyhexyl)-3-methyl-7-propylxanthin oder 1-(5-Oxohexyl)-3-methyl-7-propylxanthin (Propentofyllin) sind für die medizinische Anwendung besonders geeignet und somit bevorzugt.

Überraschenderweise eignen sich nun die Kombinationspräparate, die Gegenstand der vorliegenden Erfindung sind, ganz besonders für die klinische Therapie der obengenannten Krankheiten, weil aufgrund der überadditiven Wirkungen die in Form der erfindungsgemäßen Kombinationspräparate anzuwendenden Mengen der Kombinationsbestandteile auf solche Mengen reduziert werden können, die bei ihrer alleinigen Anwendung eine nur minimale pharmakologische Wirkung zeigen, so daß gleichzeitig Nebenwirkungen, die von diesen Arzneimitteln hervorgerufen werden, nicht mehr auftreten bzw. verringert werden. Dies ist von großer Bedeutung, weil die Prostacyclin-Analoga ohne Enolätherstrukturanteil schwerwiegende Nebenwirkungen wie arrhythmische Effekte und Tachycardie auslösen können; aber auch Kopfschmerzen, Erbrechen und Flush sind beschrieben worden. Insbesondere zeigen auch die Heteroiminoprostacycline neben vorteilhaften medizinischen Wirkungen unerwünschte Nebenwirkungen. Auch für die Xanthine sind unerwünschte klinische Symptome beschrieben worden.

Die erfindungsgemäßen Kombinationen können zur Erzielung der überadditiven antiaggregatorischen bzw. antithrombotischen Wirkung als Dosiseinheiten in Form von festen Arzneiformen, wie Kapseln (einschließlich Mikrokapseln, die im allgemeinen keine pharmazeutischen Träger enthalten), Tabletten (einschließlich Dragees und Pillen), oder Zäpfchen vorliegen, wobei bei Verwendung von Kapseln das Kapselmaterial die Funktion des Trägers wahrnehmen und der Inhalt z.B. als Pulver, Gel, Emulsion, Dispersion oder Lösung vorliegen kann. Besonders vorteilhaft und einfach ist es jedoch, orale (perorale) Formulierungen mit den beiden Wirkstoffkomponenten A) und B) herzustellen, die die berechneten Mengen der Wirkstoffe zusammmen mit jedem gewünschten pharmazeutischen Träger enthalten. Auch eine entsprechende Formulierung (Zäpfchen) für die rektale Therapie kann angewandt werden. Ebenso ist die transdermale und parenterale (intraperitoneale, intravenöse, subkutane, intramuskuläre) Injektion von Lösungen, die die erfindungsgemäßen Kombinationen enthalten, möglich.

Die Tabletten, Pillen oder Granulatkörper können nach üblichen Verfahren wie Preß-, Tauch- oder Wirbelbettverfahren, oder Kesseldragierung hergestellt werden und enthalten Trägermittel und andere übliche Hilfsstoffe, wie Gelatine, Agarose, Stärke, z. B. Kartoffel-, Mais- oder Weizenstärke, Zellulose, wie Äthylzellulose, Siliziumdioxid, verschiedene Zucker, wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Zelluloseester, oberflächenaktive Substanzen, Weichmacher, Pigmente und ähnliche Zusätze entsprechend dem Stand der Technik. Zur Herstellung der Arzneiformen kann jedes übliche Fließregulierungs-, Schmier- bzw. Gleitmittel wie Magnesiumstearat und Trennmittel verwendet werden.

Bevorzugt haben die Zubereitungen die Form von Mantel/Kern-Tabletten oder Mehrschichttabletten, wobei sich O-Acetylsalicylsäure und/oder das Xanthin im Mantel bzw. im Kern bzw. in einer Schicht befindet, während sich das Heteroiminoprostacylin im Kern bzw. im Mantel bzw. in einer anderen Schicht befindet. Die Wirkstoffkomponenten können auch in retardierter Form vorliegen, derart, daß eine gleichmäßige zeitliche Freisetzung erreicht wird. So können die Wirkstoffe beispielsweise an Ionenaustauscher oder an Metallkationen (z.B. Aluminium) gebunden oder an Retardierungsmaterial adsorbiert bzw. im Retardierungsmaterial (z.B. solcher auf Zellulose- oder Polystyrolharzbasis, z.B. Hydroxyäthylzellulose) eingeschlossen sein. Als Xanthin-Komponente eignen sich insbesondere im Handel erhältliche pharmazeutische Zubereitungen, die zeitlich verzögert, z.B. erst im Intestinaltrakt, freigesetzt werden. Eine verzögerte Freisetzung der Wirkstoffe kann auch erreicht werden, indem die betreffende Schicht bzw. das Kompartiment mit üblichen magensaftunlöslichen Überzügen versehen wird.

Die anzuwendende Dosierung ist selbstverständlich abhängig von verschiedenen Faktoren, wie dem zu behandelnden Lebewesen (d.h. Mensch oder Tier , Alter, Gewicht, allgemeiner Gesundheitszustand), der Schwere der Symptome, der zu behandelnden Erkrankung, (falls vorhanden) der Art der begleitenden Behandlung mit anderen Arzneimitteln, der Häufigkeit der Behandlung usw. Die Dosierungen werden im allgemeinen bis zu fünfmal pro Tag und vorzugsweise einmal bis dreimal pro Tag verabreicht. Die Gewichte der Einzelwirkstoffe sollen innerhalb des unten angegebenen Bereiches liegen bzw. innerhalb des für das zu behandelnde Lebewesen verträglichen, wirksamen Dosierungsbereichs.

Die geeignete Therapie mit den erfindungsgemäßen Kombinationen besteht somit z.B. in der Verabreichung von einer, zwei oder mehr, vorzugsweise 3 bis 8 Einzeldosierungen der erfindungsgemäßen Kombinationspräparate von A) je 0,05 bis 50, vorzugsweise 0,1 bis 25 und insbesondere bis 10 mg Heteroiminoprostacyclin und B) 100 bis 1200, vorzugsweise 100 bis 400 oder 600 mg Xanthinkomponente, wobei die Menge naturgemäß von der Zahl der Einzeldosierungen und auch der zu behandelnden Krankheit abhängig ist und eine Einzeldosierung z.B auch aus mehreren, gleichzeitig verabreichten Dosiseinheiten bestehen kann. Die Tagesdosis beträgt im allgemeinen höchstens 30 bis 50 mg der Komponente A und höchstens 2000 mg Xanthin, insbesondere bei oraler bzw. rektaler Verabreichung. Bei i.v.- bzw. i.p.-Verabreichung liegt die obere Tagesdosis dieser Wirkstoffe in allgemeinen um 40% bis 50% niedriger. Bei Dauerinfusionen bzw. intermittierenden Infusionen, z.B. bis zu 14 Tagen, soll eine Dosierung von 0,005 mg bis 0,01 mg Heteroiminoprostacyclin pro Minute und 0,1- 1 mg Xanthin pro Minute möglichst nicht überschritten werden, wenngleich dies bei besonders schweren Krankheitssymptomen doch vorübergehend angezeigt sein kann.

Die erfindungsgemäßen Mittel können in gleicher Weise wie bekannte antithrombotische und die Blutplättchenaggregation sowie die Metastasenwirkung inhibierende Mittel angewendet werden. In vivo-Anwendungen umfassen die Verabreichung an Menschen und Tieren, um die Bildung von Blutgerinnseln zu verhindern, wie zur Verhinderung von vorübergehenden ischämischen Anfällen und bei der Langzeitprophylaxe nach Herzinfarkten und Schlaganfällen, sowie bei Arteriosklerose und peripheren Durchblutungsstörungen (Claudicatio intermittens, Gangrän), aber auch bei der Operationsnachbehandlung zur Verhinderung postoperativer Thrombosen sowie bei der Nachbehandlung von Krebs zur Verhinderung bzw. Verminderung der Metastasenwirkung. Auch die Anwendung bei Patienten, die an Herz-Lungen-Maschinen sowie an die Nierendialyse angeschlossen sind, ist möglich, ebenso bei Patienten mit künstlichen Herzklappen, Gefäßprothesen, usw. Natürlich ist auch die Anwendung für die Schmerzstillung und Entzündungshemmung möglich.

Beispiele erfindungsgemäßer pharmazeutischer Zubereitungen

Beispiele solcher Zubereitungen für die Anwendung am Menschen (s. Tab. 1) enthalten y mg Pentoxifyllin oder andere Xanthinderivate als Reinsubstanz und/oder als handelsübliche Fertigzubereitung (Trental$^{(R)}$, Fa. Albert Roussel Pharma GmbH, Wiesbaden oder auch Rentylin$^{(R)}$, Fa. Dr. Rentschler Arzneimittel GmbH & Co., Laupheim (abgekürzt T bzw. R), oder Teile dieser Fertigzubereitungen, kombiniert mit z mg Tilsuprost. Die pharmazeutischen Träger dieser Kombinationen sind durch Erhitzen verfestigte Gele: (a) 20 Gewichtsprozent Gelatine/1 Gewichtsprozent Glycerin in Wasser sowie b) 1 Gewichtsprozent Agarose in Wasser sowie c) 10 Gewichtsprozent Äthylzellulose T50 (Hercules GmbH, Hamburg) in Aceton/ Wasser (80 : 20 Gew.%), jeweils mit und ohne 8 Gewichtsprozent eingerührtes Pentoxifyllin oder anderes Xanthinderivat oder aber handelsübliche Gelatinekapseln (für die Anwendung am Menschen und Großtieren Größe 0 (Fa. Kapsugel, Basel)). Für die Anwendung am Kleintier (Maus) werden die Bestandteile der Zubereitungen entsprechend den in Tab. 3 genannten Wirkstoffmengen reduziert bzw. kleinere Gelatinekapseln (Größe 4) verwendet.

## Methodik

### Methode 1 - Induzierte Aggregation von Humanthrombozyten in vitro

Gesund erscheinenden männlichen und weiblichen Freiwilligen, die im vorangegangenen Zeitraum von 10 Tagen keine Medikamente eingenommen haben, wird durch vorsichtige Kanülierung der Antekubitalvene Blut entnommen, das sofort mit Natriumzitrat (ad 0,38%) stabilisiert wird. Durch 15minütiges Zentrifugieren bei 140 x g erhält man im Überstand plättchenreiches Plasma (PRP), dessen Thrombozytengehalt im Bereich 2,5 - $3{,}5 \cdot 10^{+8}$ /ml (Coulter Counter) liegen soll. Die Plättchenaggregation wird optisch durch Messung der Lichttransmission im Born'schen Aggregometer verfolgt. Das Gesamtvolumen des Testansatzes beträgt 0,25 ml. Die Vorinkubationszeit mit dem Prüfpräparat beträgt 10 min bei 37 °C, die Aggregationsinduktion erfolgt sodann mit $2 \cdot 10^{-4}$ mol/lArachidonsäure oder 1,7 µg/ml Kollagen. Das Prüfpräparat wird an fünf verschiedenen Spendern getestet. Aus den jeweiligen maximalen Aggregationsamplituden werden Dosis-Wirkungskurven erstellt und graphisch die arithmetisch gemittelten $ED_{50}$ (mol/l)-Werte bestimmt. Die Messungen erfolgen im Zeitraum von 1-6 Stunden nach Blutentnahme.

### Methode 2 - Induzierte Aggregation von Thrombozyten der Maus in vivo

Weibliche Mäuse (Stamm NMRI = Naval Medical Research Institute), etwa 20-25 g Gewicht, werden per os mit den Wirkstoffen bzw. Wirkstoffkombinationen behandelt. Nach 1 Stunde erfolgt die Blutentnahme aus der Aorta unter Pentobarbitalbetäubung. Die Herstellung von PRP und die Aggregationsinduktion mit 6,8 µg/ml Kollagen oder $3{,}66 \cdot 10^{-3}$ mol/l Arachidonsäure erfolgt wie oben beschrieben (vgl. auch Thromb. Haemostasis 45 (1981) l).

### Methode 3 - Cyclo AMP-Bestimmung in Humanthrombozyten

Die Methode folgt in allen Einzelheiten der Vorschrift in Nature (London) 292 (1981) 364: Statt Kaninchenthrombozyten wurde jedoch menschliches Thrombozytenkonzentrat mit $^{3}$H-Adenin bei 20 °C für 90 min inkubiert. Das überschüssige Adenin wird, wie in Nature beschrieben, entfernt, die Wirkstoffe (s. Tab. 2) zugesetzt und erneut 5 min (37 °C) inkubiert. Das gebildete $^{3}$H-cyclo AMP wird in der bekannten Weise abgetrennt und durch Radioaktivitätsmessung quantifiziert.

**Pharmazeutische Zubereitungen** **Tab. 1**

| Beispiel | Pharmazeutischer Träger | Xanthin-Gehalt (mg) | Heteroiminoprostacyclin-Gehalt (mg) |
|---|---|---|---|
| 1 | Kapsel | 300 (T, zylindrisch gepreßt) | 0,15 (Tilsuprost) |
| 2 | Kapsel | 100 (Pentifyllin) | 0,5 (Tilsuprost) |

Pharmazeutische Zubereitungen

Tab. 1 (Forts.)

| Beispiel | Pharmazeutischer Träger | Xanthin-Gehalt (mg) | Heteroiminoprostacyclin-Gehalt (mg) |
|---|---|---|---|
| 3 | 1. Schicht: Agarose, enthaltend 470 mg Pentoxifyllin plus 0,8 mg Tilsuprost 2. Schicht: R | 470 (Pentoxifyllin) | 0,8 (Tilsuprost) |
| 4 | 1 ml wäßrige Infusionslösung | 0,015 (Pentoxifyllin) | 0,0002 (Tilsuprost) |

In Tab. 2 wird gezeigt, daß die erfindungsgemäß verwendete Komponente A (Tilsuprost) in ihrer pharmakologischen Wirkung mit dem Prostacyclin nicht vergleichbar ist.

Tab. 2

| | Hemmung der Aggregation $ED_{50}$ (mol/l) (Methode 1) | cyclo AMP-Spiegel mol/Testansatz (bei ...mol/l Wirkung) |
|---|---|---|
| Tilsuprost | $8 \cdot 10^{-9}$ | $8{,}9 \cdot 10^{-13}$ (bei $1 \cdot 10^{-9}$)<br>$9{,}8 \cdot 10^{-13}$ (bei $1 \cdot 10^{-8}$) |
| Prostacyclin | $8 \cdot 10^{-9}$ | $1{,}71 \cdot 10^{-12}$ (bei $1 \cdot 10^{-9}$)<br>$19{,}35 \cdot 10^{-12}$ (bei $1 \cdot 10^{-8}$) |
| ohne Wirkstoff | (keine Hemmung) | $9{,}0 \cdot 10^{-13}$ |

Die Ergebnisse zeigen, daß Tilsuprost und Prostacyclin äquipotent als Hemmstoffe der Aggregation von Humanthrombozyten sind. Das Experiment der cyclo AMP-Stimulierung in Humanthrombozyten durch Prostacyclin bestätigt die bereits aus Nature 292 (1981) 364 bekannten Befunde. Überraschenderweise zeigt Tilsuprost in diesem Experiment keine signifikante Stimulierung.

Die überadditive Wirkung der erfindungsgemäßen Kombinationen in menschlichen Thrombozyten wird durch die in Tab. 3 aufgeführten Ergebnisse belegt. Die Ergebnisse zeigen, daß die Hemmwirkung von Tilsuprost in der Aggregation von Humanthrombozyten (Methode 1) durch Zusatz von Xanthine überadditiv gesteigert werden kann. Die Tabelle zeigt weiterhin, daß Kombinationen aus Xanthinen der Formel III mit $R^3$ = Wasserstoff und Tilsuprost weniger ausgeprägte Wirkungen haben.

Tab. 3

| Monosubstanzen( Vergleich) (Konzentrationen in mol/l) | % Hemmung der Aggregation | Kombinationen | % Hemmung der Aggregation |
|---|---|---|---|
| | | A) Erfindungsgemäß | |
| I $1{,}9 \cdot 10^{-9}$ Tilsuprost | 5 | II + V | 93 |
| II $3{,}8 \cdot 10^{-9}$ Tilsuprost | 19 | II + VI | 84 |
| III $1{,}6 \cdot 10^{-6}$ Carbacyclin | 36 | II + VII | 95 |
| V $2{,}7 \cdot 10^{-5}$ Pentoxifyllin | 0 | B) Vergleich | |
| VI $1{,}1 \cdot 10^{-5}$ Pentifyllin | 0 | II + VIII | 45 |
| VII $2{,}9 \cdot 10^{-5}$ Propentofyllin | 0 | II + IX | 58 |
| VIII $3 \cdot 10^{-5}$ Theophyllin | 4 | | |
| IX $1{,}4 \cdot 10^{-5}$ 1-Methyl-3-isobutyl-xanthin | 5 | | |

**Patentansprüche**

**Patentansprüche für folgende Vertragsstaaten : BE, CH, DE, FR, GB, IT, LI, LU, NL, SE**

1. Kombinationspräparat, enthaltend

A) ein Heteroiminoprostacyclin der Formel I I

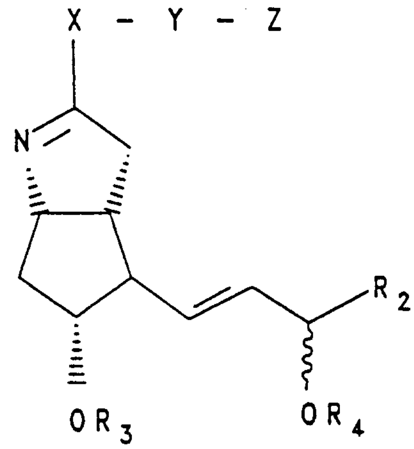

(I)

worin bedeutet

- X ein Sauerstoff- oder Schwefelatom oder eine NH-Gruppe,
- Y einen geradkettigen aliphatischen Kohlenwasserstoffrest mit 2 - 6 Kohlenstoffatomen oder einen cycloaliphatischen Kohlenwasserstoffrest mit 3 - 6 Kohlenstoffatomen im Ring, die höchstens eine Mehrfachbindung enthalten können,
- Z einen Rest der Formel $-CO_2R^1$ oder $-CH_2OH$, wobei $R^1$ Wasserstoff, einen Alkylrest mit bis zu 8 Kohlenstoffatomen, einen einfach olefinisch-ungesättigten aliphatischen Kohlenwasserstoffrest mit 3 - 8 Kohlenstoffatomen, einen cycloaliphatischen Kohlenwasserstoffrest, der 3 - 7 Kohlenstoffatome im Ring und höchstens eine Mehrfachbindung enthält, oder einen araliphatischen Kohlenwasserstoffrest mit 6 - 9 Kohlenstoffatomen oder ein pharmazeutisch verträgliches Metall- oder Ammoniumkation darstellt,
- $R^2$ einen aliphatischen Kohlenwasserstoffrest mit 4 bis 6 Kohlenstoffatomen, der höchstens eine Mehrfachbindung enthalten kann,
- $R^3$ und $R^4$ Wasserstoff oder gleiche oder verschiedene, unter neutralen oder basischen Bedingungen leicht abspaltbare Schutzgruppen,

und

B) wenigstens ein Xanthin der Formel (III) oder (IIIa),

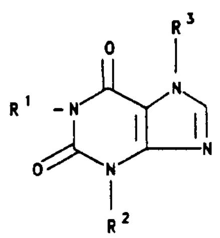

(III)

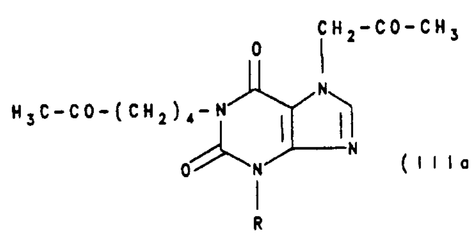

(IIIa)

worin einer der Reste $R^1$ und $R^3$ eine geradkettige Alkyl-, ($\omega$-1)-Oxoalkyl- oder ($\omega$-1)-Hydroxyalkylgruppe - die in $\omega$- oder ($\omega$-2)-Stellung eine weitere Hydroxylgruppe enthalten kann - mit 3 bis 8 C-Atomen und die beiden anderen Reste $R^2$ und $R^3$ oder $R^1$ und $R^2$ Alkylgruppen mit 1 bis 8 C-Atomen in der Position von $R^1$ bzw. $R^3$ und 1 bis 4 C-Atomen in der Position von $R^2$ darstellen und in Formel (IIIa) R für einen Alkylrest mit 1 bis 4 C-Atomen steht, oder eine Prodrug-Form der Oxoalkylxanthine der Formeln (III) oder (IIIa) oder der Hydroxyalkylxanthine der Formel (III), oder einen Metabolit eines Xanthins der Formel (III) oder (IIIa),

wobei das Gewichtsverhältnis der Komponente A zur Komponente B zwischen 1 : 8000 und 1 : 10 liegt, mit oder ohne einen pharmazeutischen Träger, zur Behandlung von Krankheiten, die durch gestörte Blutfunktionen oder Blutbestandteile, insbesondere Thrombozyten oder Erythrozyten, oder durch gestörte physiologische Wechselwirkungen zwischen den Blutbestandteilen und der Blutgefäßwand verursacht oder gekennzeichnet sind.

**2.** Kombinationspräparat gemäß Anspruch 1, dadurch gekennzeichnet, daß es als Komponente A das Heteroiminoprostacyclin die Formel V aufweist

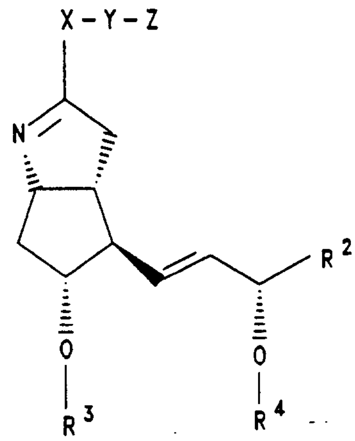

(V)

wobei X, Y, Z, $R^2$, $R^3$ oder $R^4$ die in Formel I genannte Definitionen haben, und als Komponente B das Xanthinderivat der Formel III
wobei
a) die Summe der C-Atome aller Substituenten 3 bis 10 beträgt,
b) $R^1$ oder $R^3$ ein Rest mit 6 C-Atomen ist oder
c) $R^1$ oder $R^3$ ein Rest mit 6 C-Atomen ist, der in ($\omega$-1)-Stellung mit einer Hydroxy- oder Oxogruppe substituiert ist,
enthält.

**3.** Kombinationspräparat gemäß Anspruch 1 oder 2, dadurch gekennzeichnet, daß es als Komponente A das Tilsuprost der Formel II

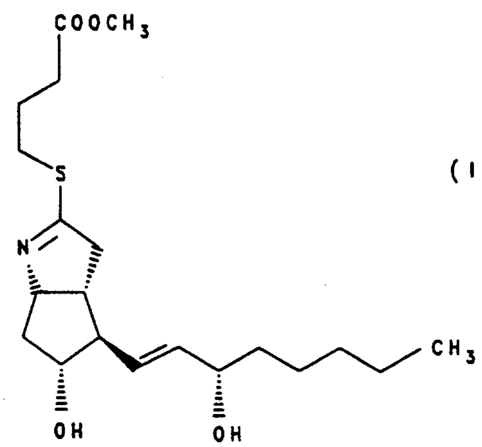

(II)

enthält.

**4.** Kombinationspräparat gemäß einem oder mehreren der Ansprüche 1 bis 3, dadurch gekennzeichnet, daß es als Komponente B 1-Hexyl-3,7-diemthylxanthin, 1-(5-Oxohexyl)-3,7-dimethylxanthin oder 1-(5-Oxohexyl)-3-methyl-7-propylxanthin enthält.

**5.** Kombinationspräparat gemäß einem oder mehreren der Ansprüche 1 bis 4, dadurch gekennzeichnet, daß das Gewichtsverhältnis der Komponente A zu der Komponente B zwischen 1 : 2000 und 1 : 20 liegt.

**6.** Kombinationspräparat gemäß Anspruch 5, dadurch gekennzeichnet, daß das Gewichtsverhältnis zwischen 1 : 500 und 1 : 50 liegt.

**7.** Kombinationspräparat gemäß einem oder mehreren der Ansprüche 1 bis 6, dadurch gekennzeichnet, daß wenigstens eine der Komponenten A und B in retardierter Form vorliegt oder in Form einer Mantel/Kern-Tablette oder Mehrschichttablette vorliegt.

**8.** Kombinationspräparat gemäß einem oder mehreren der Ansprüche 1 bis 7, dadurch gekennzeichnet, daß das Kombinationspräparat in einer oral oder rektal zu verabreichenden Dosierungseinheit vorliegt, enthaltend 0,1 mg bis 25 mg der Komponente A oder 100 mg bis 1200 mg der Komponente B.

**9.** Kombinationspräparat gemäß Anspruch 8 enthaltend 0,1 mg bis 10 mg der Komponente A oder 100 mg bis 600 mg der Komponente B.

**10.** Verwendung von Komponenten A und B gemäß einem oder mehreren der Ansprüche 1 bis 9, Komponenten A, B und C, wobei C O-Acetylsalicylsäure oder pharmazeutisch verträgliche Salze von O-Acetylsalicylsäure darstellt, oder den Komponenten A und C, wobei das Gewichtsverhältnis der Komponente A und zur Komponente B oder A zur Komponente C oder A zu den Komponenten B und C zwischen 1 : 8000 und 1 : 10 liegt,
mit oder ohne pharmazeutischen Träger, zur Herstellung von einem Arzneimittel zur Behandlung von Krankheiten, die durch gestörte Blutfunktionen oder Blutbestandteile, insbesondere Thrombozyten oder Erythrozyten, oder durch gestörte physiologische Wechselwirkungen zwischen den Blutbestandteilen und der Blutgefäßwand verursacht oder gekennzeichnet sind.

**11.** Verwendung gemäß Anspruch 10, dadurch gekennzeichnet, daß es ein Kombinationspräparat gemäß einem oder mehreren der Ansprüche 1 bis 9 enthält.

**12.** Verfahren zur Herstellung eines Kombinationspräparats gemäß einem oder mehreren der Ansprüche 1 bis 9, dadurch gekennzeichnet, daß man A) ein Heteroiminoprostacyclin der Formel (I) gemäß Anspruch 1 und B) Xanthine der Formel (III) oder (IIIa) gemäß Anspruch 1 oder Prodrug-Formen der Oxoalkylxanthine der Formeln (III) oder (IIIa) oder der Hydroxyalkylxanthine der Formel (III), oder deren Metabolite mit oder ohne pharmazeutische Träger in üblicher Weise zu einer pharmazeutischen Darbietungsform verarbeitet mit der Maßgabe, daß das Gewichtsverhältnis der Komponente A) zur Komponente B) zwischen 1 : 8000 und 1 : 10 liegt.

**Patentansprüche für folgenden Vertragsstaat : AT**

**1.** Verfahren zur Herstellung von einem Kombinationspräparat, dadurch gekennzeichnet, daß man

A) ein Heteroiminoprostacyclin der Formel I

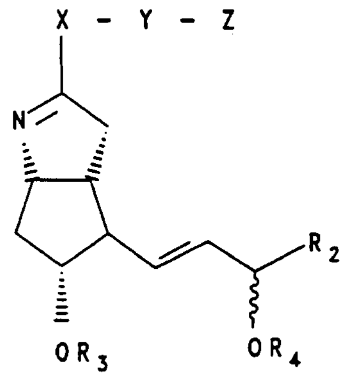

( I )

worin bedeutet

X ein Sauerstoff- oder Schwefelatom oder eine NH-Gruppe,

Y einen geradkettigen aliphatischen Kohlenwasserstoffrest mit 2 - 6 Kohlenstoffatomen oder einen cycloaliphatischen Kohlenwasserstoffrest mit 3 - 6 Kohlenstoffatomen im Ring, die höchstens eine Mehrfachbindung enthalten können,

Z einen Rest der Formel $-CO_2R^1$ oder $-CH_2OH$, wobei $R^1$ Wasserstoff, einen Alkylrest mit bis zu 8 Kohlenstoffatomen, einen einfach olefinisch-ungesättigten aliphatischen Kohlenwasserstoffrest mit 3 - 8 Kohlenstoffatomen, einen cycloaliphatischen Kohlenwasserstoffrest, der 3 - 7 Kohlenstoffatome im Ring und höchstens eine Mehrfachbindung enthält, oder einen araliphatischen Kohlenwasserstoffrest mit 6 - 9 Kohlenstoffatomen oder ein pharmazeutisch verträgliches Metall- oder Ammoniumkation darstellt,

$R^2$ einen aliphatischen Kohlenwasserstoffrest mit 4 bis 6 Kohlenstoffatomen, der höchstens eine Mehrfachbindung enthalten kann,

$R^3$ und $R^4$ Wasserstoff oder gleiche oder verschiedene, unter neutralen oder basischen Bedingungen leicht abspaltbare Schutzgruppen,

und

B) wenigstens ein Xanthin der Formel (III) bzw. (IIIa),

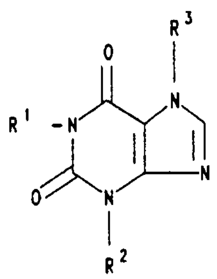

(III) bzw.

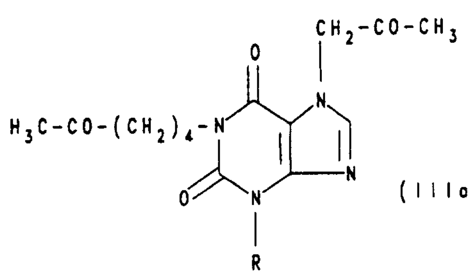

(IIIa)

worin einer der Reste $R^1$ und $R^3$ eine geradkettige Alkyl-, ($\omega$-1)-Oxoalkyl- oder ($\omega$-1)-Hydroxyalkylgruppe - die in $\omega$- oder ($\omega$-2)-Stellung eine weitere Hydroxylgruppe enthalten kann - mit 3 bis 8 C-Atomen und die beiden anderen Reste $R^2$ und $R^3$ oder $R^1$ und $R^2$ Alkylgruppen mit 1 bis 8 C-Atomen in der Position von $R^1$ bzw. $R^3$ und 1 bis 4 C-Atomen in der Position von $R^2$ darstellen und in Formel (IIIa) R für einen Alkylrest mit 1 bis 4 C-Atomen steht, oder eine Prodrug-Form der Oxoalkylxanthine der Formeln (III) oder (IIIa) oder der Hydroxyalkylxanthine der Formel (III), oder einen Metabolit eines Xanthins der Formel (III) oder (IIIa)

mit oder ohne einen pharmazeutischen Träger in üblicher Weise zu einer pharmazeutischen

Darbietungsform verarbeitet mit der Maßgabe, daß das Gewichtsverhältnis der Komponente A) zur Komponente B) zwischen 1 : 8000 und 1 : 10 liegt.

2. Verfahren nach Anspruch 1, dadurch gekennzeichnet, daß das Gewichtsverhältnis der Komponente A) zu der Komponente B) zwischen 1 : 2000 und 1 : 20, insbesondere zwischen 1 : 500 und 1 : 50 liegt.

3. Verfahren nach Anspruch 1 oder 2, dadurch gekennzeichnet, daß das Heteroiminoprostacyclin das Tilsuprost der Formel II ist

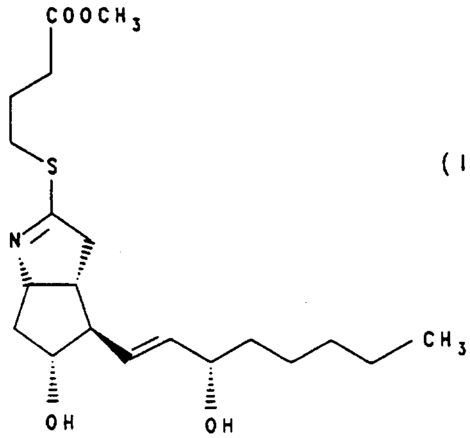

(II)

4. Verfahren nach einem oder mehreren der Ansprüche 1 bis 3, dadurch gekennzeichnet, daß ein Xanthinderivat der Formel (III) zugegen ist, bei dem die Summe der C-Atome aller Subtituenten mindestens 3 und höchstens 10 beträgt, wobei sich in der Position von $R^1$ oder $R^3$ vorzugsweise eine Gruppe mit 6 C-Atomen befindet, die bis zu einer Hydroxy- oder Oxogruppe in (ω-1)-Stellung enthalten kann.

5. Verfahren nach Anspruch 4, dadurch gekennzeichnet, daß die Xanthinverbindung 1-Hexyl-3,7-dimethylxanthin, 1-(5-Oxohexyl)-3,7-dimethylxanthin oder 1-(5-Oxohexyl)-3-methyl-7-propylxanthin ist.

6. Verfahren nach einem oder mehreren der Ansprüche 1 bis 5, dadurch gekennzeichnet, daß wenigstens eine der Komponenten A) und B) in retardierter Form vorliegt.

7. Verfahren nach einem oder mehreren der Ansprüche 1 bis 6, dadurch gekennzeichnet, daß das Kombinationspräparat in einer oral oder rektal zu verabreichenden Dosiereinheit vorliegt, vorzugsweise einer solchen, die A) 0,1 bis 25 insbesondere bis 10 mg Heteroiminoprostacyclin und B) 100 bis 1200, vorzugsweise 100 bis 600 mg Xanthinkomponente enthält.

8. Verfahren nach einem oder mehreren der Ansprüche 1 bis 7, dadurch gekennzeichnet, daß das Präparat oder Mittel in Form einer Mantel/Kern-Tablette oder Mehrschichttablette vorliegt.

9. Verfahren nach einem oder mehreren der Ansprüche 1 bis 8, dadurch gekennzeichnet, daß das Heteroiminoprostacyclin die Formel V aufweist

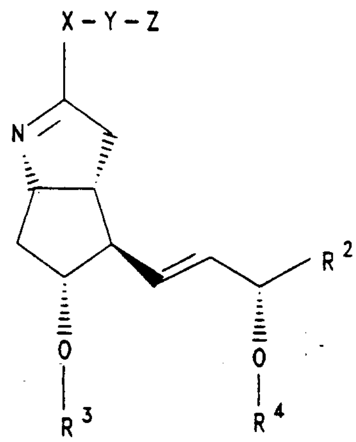

(V)

wobei X, Y, Z, $R^2$, $R^3$ oder $R^4$ die in Formel I genannte Bedeutung haben.

**Claims**

**Claims for the following Contracting States : BE, CH, DE, FR, GB, IT, LI, LU, NL, SE**

**1.** A combination product containing

A) a heteroiminoprostacyclin of the formula I

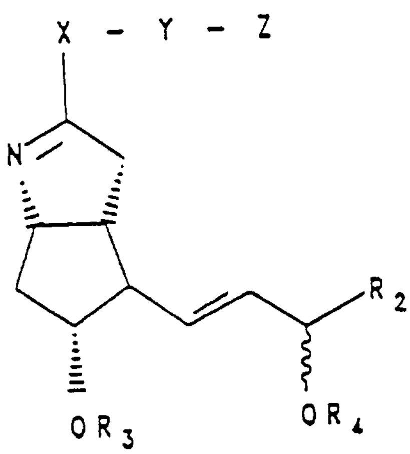

(I)

in which

| | |
|---|---|
| X | denotes an oxygen or sulfur atom or an NH group, |
| Y | denotes a straight-chain aliphatic hydrocarbon radical having 2-6 carbon atoms or a cycloaliphatic hydrocarbon radical having 3-6 carbon atoms in the ring, which radicals can contain not more than one multiple bond, |
| Z | denotes a radical of the formula $-CO_2R^1$ or $-CH_2OH$, $R^1$ denoting hydrogen, an alkyl radical having up to 8 carbon atoms, a singly olefinically unsaturated aliphatic hydrocarbon radical having 3-8 carbon atoms, a cycloaliphatic hydrocarbon radical which has 3-7 carbon atoms in the ring and not more than one multiple bond, or an araliphatic hydrocarbon radical having 6-9 carbon atoms or a pharmaceutically tolerated metal or ammonium cation, |
| $R^2$ | denotes an aliphatic hydrocarbon radical which has 4 to 6 carbon atoms and which can contain not more than one multiple bond, and |
| $R^3$ and $R^4$ | denote hydrogen or identical or different protective groups which can readily be eliminated under neutral or basic conditions, |

and

B) at least one xanthine of the formula (III) or (IIIa)

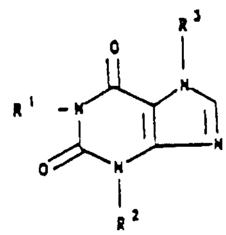
(III)

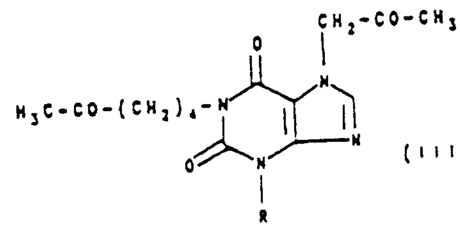
(IIIa)

in which one of the radicals $R^1$ and $R^3$ represents a straight-chain alkyl, ($\omega$-1)-oxoalkyl or ($\omega$-1)-hydroxyalkyl group - which can contain another hydroxyl group in the $\omega$ or ($\omega$-2) position - having 3 to 8 carbon atoms, and the two other radicals $R^2$ and $R^3$ or $R^1$ and $R^2$ represent alkyl groups having 1 to 8 carbon atoms in the position of $R^1$ or $R^3$ and 1 to 4 carbon atoms in the position of $R^2$, and R in formula (IIIa) represents an alkyl radical having 1 to 4 carbon atoms, or a prodrug form of the oxoalkylxanthines of the formulae (III) or (IIIa) or of the hydroxyalkylxanthines of the formula (III), or a metabolite of a xanthine of the formula (III) or (IIIa),
and the ratio by weight of the component A to the component B being between 1:8,000 and 1:10, with or without a pharmaceutical vehicle, for the treatment of diseases which are caused or characterized by defects in blood functions or blood constituents, in particular platelets and erythrocytes, or by defects in the physiological interactions between the blood constituents and the blood vessel wall.

**2.** A combination product as claimed in claim 1, which contains as component A the heteroiminoprostacyclin which has formula V

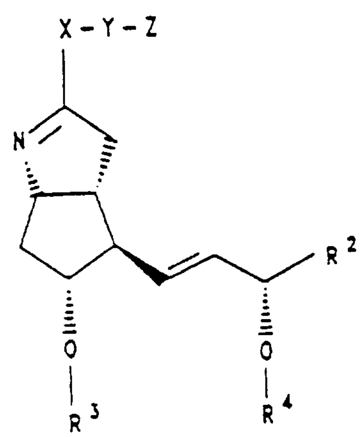
(V)

where X, Y, Z, $R^2$, $R^3$ or $R^4$ have the definitions specified in formula I, and as component B the xanthine derivative of the formula III,
where

a) the total of the carbon atoms in all the substituents is 3 to 10,
b) $R^1$ or $R^3$ is a radical having 6 carbon atoms, or
c) $R^1$ or $R^3$ is a radical which has 6 carbon atoms and is substituted by a hydroxyl or oxo group in the ($\omega$-1) position.

**3.** A combination product as claimed in claim 1 or 2, which contains as component A tilsuprost of the formula II

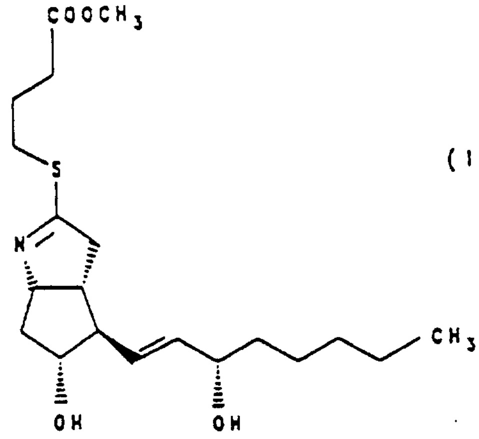

(II)

4. A combination product as claimed in one or more of claims 1 to 3, which contains as component B 1-hexyl-3,7-dimethylxanthine, 1-(5-oxohexyl)-3,7-dimethylxanthine or 1-(5-oxohexyl)-3-methyl-7-propylxanthine.

5. A combination product as claimed in one or more of claims 1 to 4, wherein the ratio by weight of component A to component B is between 1:2,000 and 1:20.

6. A combination product as claimed in claim 5, wherein the ratio by weight is between 1:500 and 1:50.

7. A combination product as claimed in one or more of claims 1 to 6, wherein at least one of components A and B is present in controlled-release form or in the form of a coating/core tablet or multilayer tablet.

8. A combination product as claimed in one or more of claims 1 to 7, wherein the combination product is in the form of a dosage unit which is to be administered orally or rectally and contains 0.1 mg to 25 mg of component A or 100 mg to 1,200 mg of component B.

9. A combination product as claimed in claim 8 containing 0.1 mg to 10 mg of component A or 100 mg to 600 mg of component B.

10. The use of components A and B as claimed in one or more of claims 1 to 9, components A, B and C, where C represents O-acetylsalicylic acid or pharmaceutically tolerated salts of O-acetylsalicylic acid, or of components A and C, where the ratio by weight of component A to component B or A to component C or A to components B and C is between 1:8,000 and 1:10, with or without pharmaceutical vehicles, for the preparation of a pharmaceutical for the treatment of diseases which are caused or characterized by defects in blood functions or blood constituents, in particular platelets and erythrocytes, or by defects in the physiological interactions between the blood constituents and the blood vessel wall.

11. The use as claimed in claim 10, wherein it contains a combination product as claimed in one or more of claims 1 to 9.

12. A process for the preparation of a combination product as claimed in one or more of claims 1 to 9, which comprises processing A) a heteroiminoprostacyclin of the formula (I) as claimed in claim 1 and B) xanthines of the formula (III) or (IIIa) as claimed in claim 1 or prodrug forms of the oxoalkylxanthines of the formulae (III) or (IIIa) or of the hydroxyalkylxanthines of the formula (III), or their metabolites, with or without pharmaceutical vehicles, in a conventional way to a pharmaceutical presentation, with the proviso that the ratio by weight of component A) to component B) is between 1:8,000 and 1:10.

**Claims for the following Contracting State : AT**

**1.** A process for the preparation of a combination product, which comprises processing

A) a heteroiminoprostacyclin of the formula I

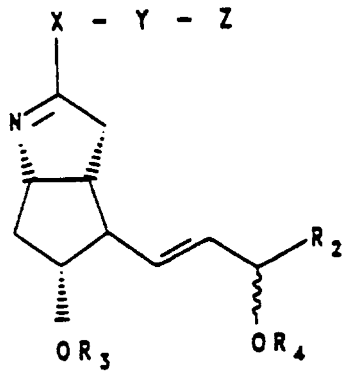

(I)

in which

| | |
|---|---|
| X | denotes an oxygen or sulfur atom or an NH group, |
| Y | denotes a straight-chain aliphatic hydrocarbon radical having 2-6 carbon atoms or a cycloaliphatic hydrocarbon radical having 3-6 carbon atoms in the ring, which radicals can contain not more than one multiple bond, |
| Z | denotes a radical of the formula $-CO_2R^1$ or $-CH_2OH$, $R^1$ denoting hydrogen, an alkyl radical having up to 8 carbon atoms, a singly olefinically unsaturated aliphatic hydrocarbon radical having 3-8 carbon atoms, a cycloaliphatic hydrocarbon radical which has 3-7 carbon atoms in the ring and not more than one multiple bond, or an araliphatic hydrocarbon radical having 6-9 carbon atoms or a pharmaceutically tolerated metal or ammonium cation, |
| $R^2$ | denotes an aliphatic hydrocarbon radical which has 4 to 6 carbon atoms and which can contain not more than one multiple bond, and |
| $R^3$ and $R^4$ | denote hydrogen or identical or different protective groups which can readily be eliminated under neutral or basic conditions, |

and

B) at least one xanthine of the formula (III) or (IIIa)

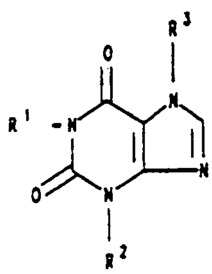

(III) bzw.

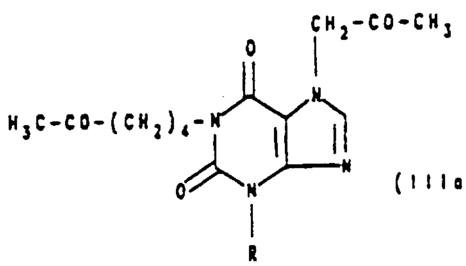

(IIIa)

in which one of the radicals $R^1$ and $R^3$ represents a straight-chain alkyl, ($\omega$-1)-oxoalkyl or ($\omega$-1)-hydroxyalkyl group - which can contain another hydroxyl group in the $\omega$ or ($\omega$-2) position - having 3 to 8 carbon atoms, and the two other radicals $R^2$ and $R^3$ or $R^1$ and $R^2$ represent alkyl groups having 1 to 8 carbon atoms in the position of $R^1$ or $R^3$ and 1 to 4 carbon atoms in the position of $R^2$, and R in formula (IIIa) represents an alkyl radical having 1 to 4 carbon atoms, or a prodrug form of the oxoalkylxanthines of the formulae (III) or (IIIa) or of the hydroxyalkylxanthines of the formula (III), or a metabolite of a xanthine of the formula (III) or (IIIa),

with or without a pharmaceutical vehicle, in a conventional way to a pharmaceutical presentation, with the proviso that the ratio by weight of component A) to component B) is between 1:8,000 and 1:10.

**2.** The process as claimed in claim 1, wherein the ratio by weight of component A) to component B) is between 1:2,000 and 1:20, in particular between 1:500 and 1:50.

**3.** The process as claimed in claim 1 or 2, wherein the heteroiminoprostacyclin is tilsuprost of the formula II

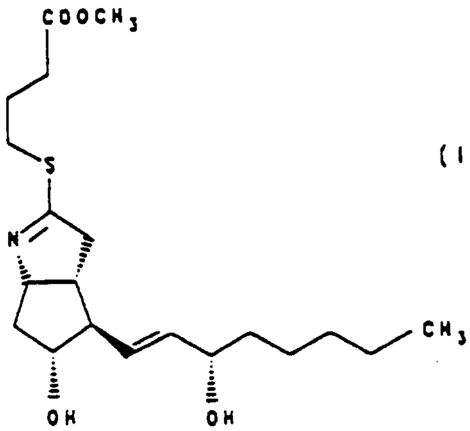

(II)

**4.** The process as claimed in one or more of claims 1 to 3, wherein a xanthine derivative of the formula (III) is present, where the total of the carbon atoms in all the substituents is at least 3 and at most 10, there preferably being present in the position of $R^1$ or $R^3$ a group which has 6 carbon atoms and can contain up to one hydroxyl or oxo group in the ($\omega$-1) position.

**5.** The process as claimed in claim 4, wherein the xanthine compound is 1-hexyl-3,7-dimethylxanthine, 1-(5-oxohexyl)-3,7-dimethylxanthine or 1-(5-oxo-hexyl)-3-methyl-7-propylxanthine.

**6.** The process as claimed in one or more of claims 1 to 5, wherein at least one of the components A) and B) is present in controlled-release form.

**7.** The process as claimed in one or more of claims 1 to 6, wherein the combination product is in the form of a dosage unit to be administered orally or rectally, preferably one which contains A) 0.1 to 25, in particular up to 10, mg of heteroiminoprostacyclin and B) 100 to 1,200, preferably 100 to 600, mg of xanthine component.

**8.** The process as claimed in one or more of claims 1 to 7, wherein the product or agent is in the form of a coating/core tablet or multilayer tablet.

**9.** The process as claimed in one or more of claims 1 to 8, wherein the heteroiminoprostacyclin has the formula V

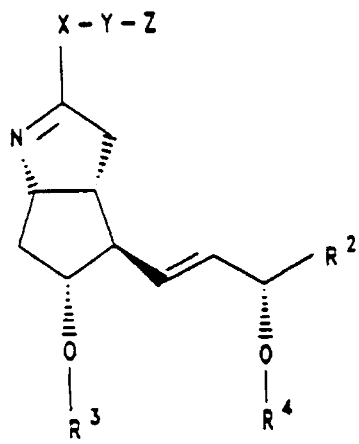

(V)

where X, Y, Z, $R^2$, $R^3$ or $R^4$ have the meaning specified in formula I.

**Revendications**

**Revendications pour les Etats contractants suivants : BE, CH, DE, FR, GB, IT, LI, LU, NL, SE**

**1.** Préparation combinée, contenant
A) une hétéroiminoprostacycline de formule I

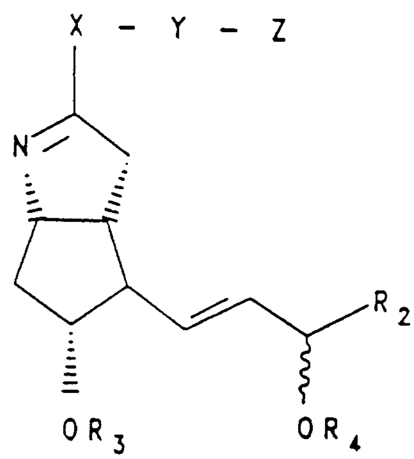

(I)

où
X représente un atome d'oxygène ou de soufre, ou un groupe NH,
Y représente un radical d'hydrocarbure aliphatique à chaîne droite ayant de 2 à 6 atomes de carbone ou un radical d'hydrocarbure cycloaliphatique ayant de 3 à 6 atomes de carbone dans le cycle, qui peuvent contenir au plus une liaison multiple,
Z représente un radical de formule $-CO_2R^1$ ou $-CH_2OH$, $R^1$ représentant un atome d'hydrogène, un radical alkyle ayant jusqu'à 8 atomes de carbone, un radical d'hydrocarbure aliphatique à insaturation oléfinique unique ayant de 3 à 8 atomes de carbone, un radical d'hydrocarbure cycloaliphatique contenant de 3 à 7 atomes de carbone dans le cycle et au plus une liaison multiple, ou un radical d'hydrocarbure arylaliphatique ayant de 6 à 9 atomes de carbone ou un cation ammonium ou un cation métallique pharmaceutiquement acceptable,
$R^2$ représente un radical d'hydrocarbure aliphatique ayant de 4 à 6 atomes de carbone qui peut contenir au plus une liaison multiple,
$R^3$ et $R^4$ représentent un atome d'hydrogène ou des groupes protecteurs, identiques ou différents, facilement séparables dans des conditions neutres ou basiques,
et

B) au moins une xanthine de formule (III) ou (IIIa),

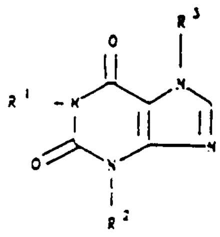
(III)

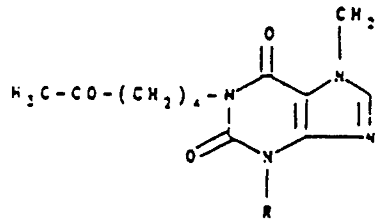
(IIIa)

où un des radicaux $R^1$ et $R^3$ représente un groupe alkyle, ($\omega$-1)-oxoalkyle ou ($\omega$-1)-hydroxyalkyle à chaîne droite - ledit ($\omega$-1)-hydroxyalkyle pouvant contenir un groupe hydroxyle supplémentaire en position $\omega$ ou ($\omega$-2) - ayant de 3 à 8 atomes de carbone, et les deux autres radicaux $R^2$ et $R^3$, ou $R^1$ et $R^2$, représentent des groupes alkyle ayant de 1 à 8 atomes de carbone dans le cas de $R^1$ et $R^3$, et de 1 à 4 atomes de carbone dans le cas de $R^2$, et, dans la formule (IIIa), R représente un radical alkyle ayant de 1 à 4 atomes de carbone, ou une forme prémédicamenteuse des oxoalkylxanthines de formules (III) ou (IIIa) ou des hydroxyalkylxanthines de formule (III), ou un métabolite d'une xanthine de formule (III) ou (IIIa),
le rapport du composant A au composant B étant compris, en poids, entre 1:8000 et 1:10, avec ou sans un véhicule pharmaceutique, pour traiter des maladies caractérisées ou provoquées par une perturbation des fonctions sanguines ou des constituants sanguins, en particulier, des thrombocytes ou des érythrocytes, ou par une perturbation des interactions physiologiques entre les constituants sanguins et la paroi des vaisseaux sanguins.

**2.** Préparation combinée selon la revendication 1, caractérisée en ce qu'elle contient, comme composant A, l'hétéroiminoprostacycline qui présente la formule V

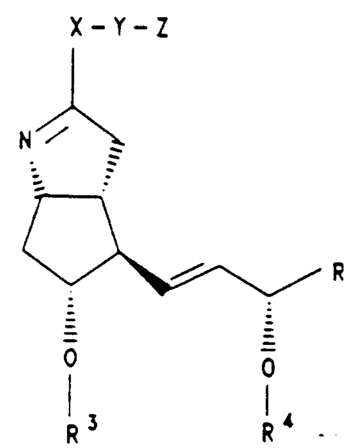
(V)

où X, Y, Z, $R^2$, $R^3$ ou $R^4$ ont les définitions citées dans la formule I,
et, comme composant B, le dérivé de xanthine de formule III, pour lequel
a) la somme des atomes de carbone de tous les substituants vaut de 3 à 10,
b) $R^1$ ou $R^3$ est un radical ayant 6 atomes de carbone, ou
c) $R^1$ ou $R^3$ est un radical ayant 6 atomes de carbone, substitué par un groupe hydroxy ou oxo en position ($\omega$-1).

**3.** Préparation combinée selon la revendication 1 ou 2, caractérisée en ce qu'elle contient, comme composant A, le tilsuprost de formule II

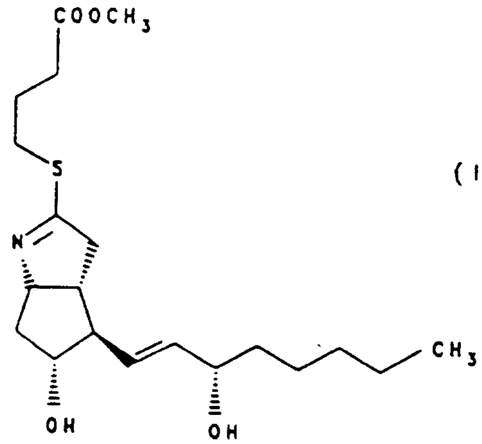

( I I )

4. Préparation combinée selon une ou plusieurs des revendications 1 à 3, caractérisée en ce qu'elle contient, comme composant B, de la 1-hexyl-3,7-diméthylxanthine, de la 1-(5-oxohexyl)-3,7-diméthylxanthine ou de la 1-(5-oxohexyl)-3-méthyl-7-propylxanthine.

5. Préparation combinée selon une ou plusieurs des revendications 1 à 4, caractérisée en ce que le rapport du composant A au composant B est compris, en poids, entre 1:2000 et 1:20.

6. Préparation combinée selon la revendication 5, caractérisée en ce que le rapport est compris, en poids, entre 1:500 et 1:50.

7. Préparation combinée selon une ou plusieurs des revendications 1 à 6, caractérisée en ce qu'au moins un des composants A et B existe sous forme retardée ou se présente sous la forme d'un comprimé noyau/enveloppe ou d'un comprimé stratifié.

8. Préparation combinée selon une ou plusieurs des revendications 1 à 7, caractérisée en ce que la préparation combinée se présente sous la forme d'une dose unitaire à administrer par voie orale ou rectale, contenant de 0,1 mg à 25 mg du composant A ou de 100 mg à 1200 mg du composant B.

9. Préparation combinée selon la revendication 8, contenant de 0,1 mg à 10 mg du composant A ou de 100 mg à 600 mg du composant B.

10. Utilisation des composants A et B selon une ou plusieurs des revendications 1 à 9, des composants A, B et C, C représentant l'acide o-acétylsalicylique ou des sels pharmaceutiquement acceptables de l'acide o-acétylsalicylique, ou des composants A et C, le rapport du composant A au composant B, ou du composant A au composant C, ou du composant A aux composants B et C étant compris, en poids, entre 1:8000 et 1:10,
avec ou sans véhicule pharmaceutique, pour préparer un médicament destiné au traitement de maladies caractérisées ou provoquées par une perturbation des fonctions sanguines ou des constituants sanguins, en particulier, des thrombocytes ou des érythrocytes, ou par une perturbation des interactions physiologiques entre les constituants sanguins et la paroi des vaisseaux sanguins.

11. Utilisation selon la revendication 10, caractérisée en ce qu'elle comporte une préparation combinée selon une ou plusieurs des revendications 1 à 9.

12. Procédé pour fabriquer une préparation combinée selon une ou plusieurs des revendications 1 à 9, caractérisé en ce que l'on traite, de manière connue en soi, A) une hétéroiminoprostacycline de formule (I) selon la revendication 1 et B) des xanthines de formule (III) ou (IIIa) selon la revendication 1 ou des formes prémédicamenteuses des oxoalkylxanthines de formules (III) ou (IIIa) ou des hydroxyalkylxanthines de formule (III), ou des métabolites de celles-ci, avec ou sans véhicule pharmaceutique, jusqu'à obtenir une forme de présentation pharmaceutique, en prenant soin que le rapport du composant A) au composant B) soit compris, en poids, entre 1:8000 et 1:10.

**Revendications pour l'Etat contractant suivant : AT**

1. Procédé pour fabriquer une préparation combinée, caractérisé en ce que l'on traite, de manière connue en soi,

A) une hétéroiminoprostacycline de formule I

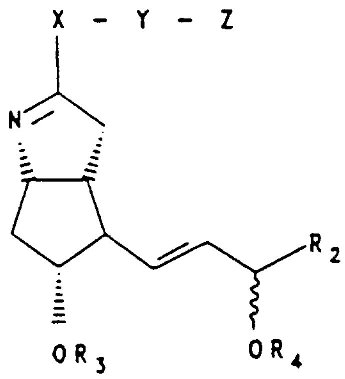

( I )

où

X représente un atome d'oxygène ou de soufre, ou un groupe NH,

Y représente un radical d'hydrocarbure aliphatique à chaîne droite ayant de 2 à 6 atomes de carbone ou un radical d'hydrocarbure cycloaliphatique ayant de 3 à 6 atomes de carbone dans le cycle, qui peuvent contenir au plus une liaison multiple,

Z représente un radical de formule $-CO_2R^1$ ou $-CH_2OH$, $R^1$ représentant un atome d'hydrogène, un radical alkyle ayant jusqu'à 8 atomes de carbone, un radical d'hydrocarbure aliphatique à insaturation oléfinique unique ayant de 3 à 8 atomes de carbone, un radical d'hydrocarbure cycloaliphatique contenant de 3 à 7 atomes de carbone dans le cycle et au plus une liaison multiple, ou un radical d'hydrocarbure arylaliphatique ayant de 6 à 9 atomes de carbone ou un cation ammonium ou un cation métallique pharmaceutiquement acceptable,

$R^2$ représente un radical d'hydrocarbure aliphatique ayant de 4 à 6 atomes de carbone qui peut contenir au plus une liaison multiple,

$R^3$ et $R^4$ représentent un atome d'hydrogène ou des groupes protecteurs, identiques ou différents, facilement séparables dans des conditions neutres ou basiques,

et

B) au moins une xanthine de formule (III) ou (IIIa),

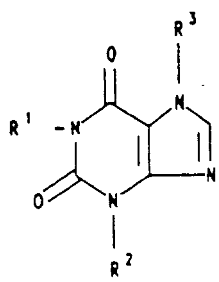

(III) bzw.

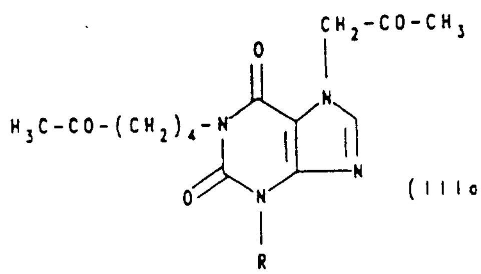

(IIIa)

où un des radicaux $R^1$ et $R^3$ représentent un groupe alkyle, (ω-1)-oxoalkyle ou (ω-1)-hydroxyalkyle à chaîne droite - ledit (ω-1)-hydroxyalkyle pouvant contenir un groupe hydroxyle supplémentaire en position ω ou (ω-2) - ayant de 3 à 8 atomes de carbone, et les deux autres radicaux $R^2$ et $R^3$, ou $R^1$ et $R^2$, représentent des groupes alkyle ayant de 1 à 8 atomes de carbone dans le cas de $R^1$ et $R^3$, et de 1 à 4 atomes de carbone dans le cas de $R^2$, et, dans la formule (IIIa), R représente un radical alkyle ayant de 1 à 4 atomes de carbone, ou une forme prémédicamenteuse des oxoalkylxanthines de formules (III) ou (IIIa) ou des hydroxyalkylxanthines de formule (III), ou un métabolite d'une xanthine de formule (III) ou (IIIa),

avec ou sans véhicule pharmaceutique, jusqu'à obtenir une forme de présentation pharmaceutique, en prenant soin que le rapport du composant A) au composant B) soit compris, en poids, entre 1:8000 et 1:10.

**2.** Procédé selon la revendication 1, caractérisé en ce que le rapport du composant A au composant B est compris, en poids, entre 1:2000 et 1:20, en particulier, entre 1:500 et 1:50.

**3.** Procédé selon la revendication 1 ou 2, caractérisé en ce que l'hétéroiminoprostacycline est le tilsuprost de formule II

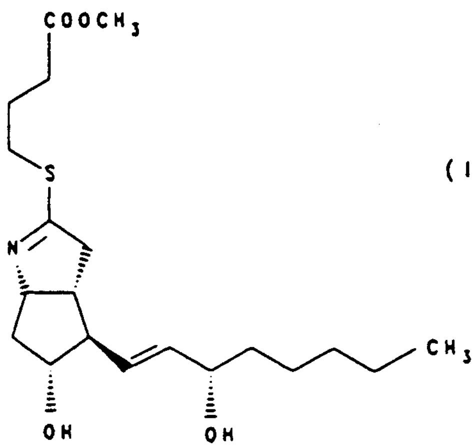

(II)

**4.** Procédé selon une ou plusieurs des revendications 1 à 3, caractérisé en ce qu'un dérivé de xanthine de formule (III) est présent, dans lequel la somme des atomes de carbone de tous les substituants vaut au moins 3 et au plus 10, la position de $R^1$ ou $R^3$ étant occupée, de préférence, par un groupe ayant 6 atomes de carbone qui peut contenir un groupe hydroxy ou oxo en position ($\omega$-1).

**5.** Procédé selon la revendication 4, caractérisé en ce que le composé de xanthine est la 1-hexyl-3,7-diméthylxanthine, la 1-(5-oxohexyl)-3,7-diméthylxanthine ou la 1-(5-oxohexyl)-3-méthyl-7-propylxanthine.

**6.** Procédé selon une ou plusieurs des revendications 1 à 5, caractérisé en ce qu'au moins un des composants A) et B) existe sous forme retardée.

**7.** Procédé selon une ou plusieurs des revendications 1 à 6, caractérisé en ce que la préparation combinée se présente sous la forme d'une dose unitaire à administrer par voie orale ou rectale, laquelle dose unitaire contient, de préférence, A) de 0,1 mg à 25 mg, en particulier, jusqu'à 10 mg d'hétéroiminoprostacycline et B) de 100 mg à 1200 mg, de préférence, de 100 mg à 600 mg de composant xanthine.

**8.** Procédé selon une ou plusieurs des revendications 1 à 7, caractérisé en ce que la préparation ou l'agent se présente sous la forme d'un comprimé noyau/enveloppe ou d'un comprimé stratifié.

**9.** Procédé selon une ou plusieurs des revendications 1 à 8, caractérisé en ce que l'hétéroiminoprostacycline présente la formule V

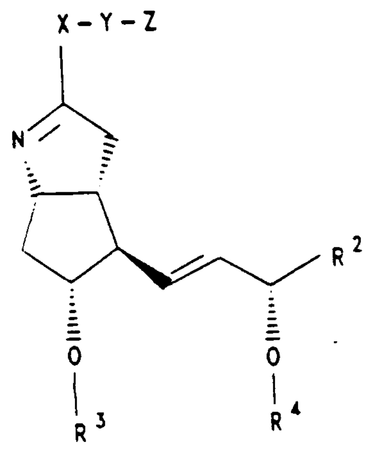

(V)

où X, Y, Z, $R^2$, $R^3$ ou $R^4$ ont la signification citée dans la formule I.